# EUROPEAN PATENT SPECIFICATION

(11) **EP 3 763 353 B1**
(45) Date of publication and mention of the grant of the patent: **24.01.2024**
(21) Application number: 18908499.9
(22) Date of filing: 08.03.2018
(51) Int. Cl.: A61K 8/34, A61Q 19/00

(54) **METHOD FOR IMPROVING WATER SOLUBILITY OF SPARINGLY-SOLUBLE COMPONENT**
VERFAHREN ZUR VERBESSERUNG DER WASSERLÖSLICHKEIT EINER SPÄRLICH LÖSLICHEN KOMPONENTE
PROCÉDÉ POUR AMÉLIORER LA SOLUBILITÉ DANS L'EAU D'UN COMPOSANT MODÉRÉMENT SOLUBLE

(43) Date of publication of application: 13.01.2021
(73) Proprietor: ADEKA CORPORATION, Arakawa-ku Tokyo 116-8554 (JP)
(72) Inventor: INOUE, Takahiro, Tokyo 116-8554 (JP); SUZUKI, Hiroshi, Tokyo 116-8554 (JP); TSUSHIMA, Yasuhiro, Tokyo 116-8554 (JP)
(74) Representative: Kador & Partner Part mbB
(86) International application number: PCT/JP2018/008973
(87) International publication number: WO 2019/171529

(56) References cited:
- EP-A1- 2 730 271
- WO-A2-2005/018582
- JP-A- H11 193 208
- JP-A- H11 322 591
- JP-A- 2003 176 210
- JP-A- 2011 173 808
- PILLAI R ET AL: "1,2-Pentanediol - a Multifunctional Ingredient for Personal Care Applications", SOFW JOURNAL, VERLAG FUER CHEMISCHE INDUSTRIE H. ZIOLKOWSKY GMBH, DE, vol. 131, no. 6, 1 June 2005 (2005-06-01), pages 12-22, XP002697177, ISSN: 0942-7694

## Description

### Technical Field

This invention relates to a method for improving the solubility of a sparingly water-soluble component in water, and also relates to a method for producing a water-based cosmetic product, the production method including a step for improving the solubility of the sparingly water-soluble component.

### Background Art

Cosmetic products are broadly classified into water-based cosmetic products that use water as a dispersion medium and contain a large amount of water and oil-based cosmetic products that use an oily component as a dispersion medium and contain a large amount of the oily component. Of these, there is high demand for water-based cosmetic products, which are preferred from the perspectives of exhibiting little stickiness and exhibiting good safety and feeling of use, and techniques for improving and enhancing the functionality of water-based cosmetic products will be required in the future as extremely important techniques.

A variety of additives are generally used in order to impart water-based cosmetic products with higher functionality. Examples of additives used in water-based cosmetic products include higher alcohols, hydrocarbons, higher fatty acids, vitamins, antioxidants, UV absorbers, preservatives, antibacterial/antiseptic agents, cosmetic components, fragrances and a variety of extracts, and a variety of other additives are also blended according to need. Some of these additives are highly water-soluble and some are highly oil-soluble. It was easy to blend highly water-soluble additives in water-based cosmetic products, but it was also necessary to blend highly oil-soluble additives in some cases, and such features required innovation.

In order to impart water-based cosmetic products with a variety of functions, use of surfactants such as emulsifying agents and solubilizing agents is an ordinary method for blending highly oil-soluble additives in water-based cosmetic products. For example, Patent Document 1 discloses a water-based liquid cosmetic product that contains (a) 10 to 40 mass% of a moisturizing agent, (b) 0.01 to 3 mass% of an oil component, (c) 0.01 to 5 mass% of a hydrophilic surfactant and (d) 0.001 to 0.3 mass% of a poly(acrylic acid) or a metal salt thereof. Patent Document 2 discloses a water-based cosmetic product that contains (A) an alkyl ethylene oxide surfactant having an HLB of 8 to 13, (B) 10 to 40 mass% of an oily component and (C) water. In addition, Patent Document 3 discloses a transparent water-based cosmetic product obtained by dissolving an oil-soluble component [component (a)] in phenoxyethanol [component (b)] and then blending the thus obtained solution in a surfactant [component (c)] and water [component (d)]. Patent document 4 discloses cosmetic and dermatological compositions containing a steroid hormone or anti-inflammatory agent and pentylene glycol. Patent document 5 discloses a non-therapeutic aqueous composition for topical application, comprising(a) at least one solid active agent having at 20 °C a solubility in water (20° dH) of less than 5 g/L; (b) at least one 1,2-alkandiol having 4 to 12 carbon atoms; and (c) at least one aliphatic alcohol having 2 to 4 carbon atoms. Patent document 6 discloses studies that exemplify the multifunctional aspects of 1,2-Pentanediol.
Patent document 7 relates to a composition for external use, virtually not containing any surfactant by applying a solubilizing means which is settled to solubilize in an aqueous phase a perfume difficultly soluble in water. Patent document 8 discloses a cosmetic that is highly safe to the human body and highly antimicrobial with a used amount of paraben suppressed low or without using paraben.

However, emulsifying agents and solubilizing agents are substances that are added in order to mix water or water-based components with oils or oil-soluble components, that is, components which inherently cannot be mixed. Therefore, if these components are blended and used in water-based cosmetic products, natural oil-soluble components, such as sebum present at the surface of human skin, are also emulsified or solubilized in some cases, and this can lead to skin problems. Therefore, it was desirable to use less or no emulsifying agent or solubilizing agent in some cases, depending on the mode of use a water-based cosmetic product.

### Citation List

### Patent Documents

[Patent Document 1] Japanese Patent Application Publication No. 2013-147434
[Patent Document 2] Japanese Translation of PCT Application No. 2011-001359
[Patent Document 3] Japanese Patent Application Publication No. 2008-195676
[Patent Document 4] WO 2005/018582
[Patent document 5] EP 2 730 271 A1
[Patent Document 6] Pillai R. et al. 1,2-Pentanediol - a Multifunctional Ingredient for Personal Care Applications. SOFW JOURNAL, June 2005 , vol. 131, no. 6, pages 12-22,
[Patent Document 7] JP H11 193208 A
[Patent Document 8] JP 2011 173808 A

### Summary of Invention

### Technical Problem

In embodiments in which use of emulsifying agents or solubilizing agents is restricted, it is possible to blend oil-soluble components in water-based cosmetic products and impart the water-based cosmetic products with high functionality, as shown in the background art disclosed in Patent Documents 1 to 3. However, in embodiments in which use of emulsifying agents or solubilizing agents is not desirable, it is extremely difficult to impart water-based cosmetic products with high functionality by means of oil-soluble components, that is to say, components that hardly dissolve in water (sparingly water-soluble components).

Therefore, the problem to be solved by this invention is to provide a method for improving the solubility of a sparingly water-soluble component. In cases where said method is used to produce a water-based cosmetic product, the problem to be solved by this invention is also to provide a production method in which a sparingly water-soluble component is blended in a water-based cosmetic product using less or no commonly used emulsifying agents or solubilizing agents for cosmetic products.

### Solution to Problem

The inventors of this invention completed this invention as a result of diligent research. That is, this invention is a method for improving the solubility of a sparingly water-soluble component, the method comprising blending a compound represented by general formula (1) below with the sparingly water-soluble component in the presence of water, 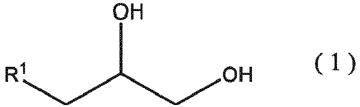 wherein R¹ denotes a group represented by general formula (2); 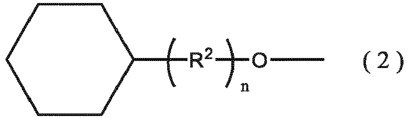 wherein R² denotes an alkylene group having 1 to 3 carbon atoms, and n represents a number that is 0 or 1 wherein the sparingly water-soluble component has a solubility in water of 3[g/100g water] or less, and wherein the sparingly water-soluble component is one or more components selected from the group consisting of sparingly water-soluble antibacterial/ antiseptic agents, sparingly water-soluble antioxidants, sparingly water-soluble vitamins, sparingly water-soluble UV absorbers, sparingly water-soluble fragrances, sparingly water-soluble cosmetic components and sparingly water-soluble vegetable oils

### Advantageous Effects of Invention

By using this invention, it is expected that it is possible to improve the solubility in water of a sparingly water-soluble component and produce a water-based cosmetic product using less or no emulsifying agent or solubilizing agent.

### Description of Embodiments

This invention is a method for improving the solubility of a sparingly water-soluble component and, more specifically, is a method for improving the solubility of a sparingly water-soluble component, the production method being characterized by blending a compound represented by general formula (1) below with the sparingly water-soluble component in the presence of water. Moreover, the method of this invention is capable of improving the solubility of a sparingly water-soluble component without using a surfactant such as an emulsifying agent or a solubilizing agent.

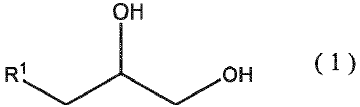

In the formula, R¹ denotes a group represented by general formula (2) :

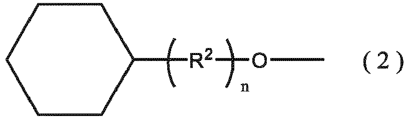

In the formula, R² denotes an alkylene group having 1 to 3 carbon atoms, and n represents a number that is 0 or 1.

In general formula (1), R¹ denotes a group represented by general formula (2)

In general formula (2), R² denotes an alkylene group having 1 to 3 carbon atoms, and specific examples thereof include a methylene group, an ethylene group, a propylene group and an isopropylene group. Of these, a methylene group or an ethylene group is preferred from the perspective of ease of preparation and procurement of raw materials.
n denotes a number that is 0 or 1, and it is preferable for n to be 0 from the perspective of being able to obtain a compound that readily achieves the advantageous effect of this invention.

A compound represented by general formula (1) can be procured by directly producing a compound represented by general formula (1) or by purchasing a commercially available product.

Methods for producing a compound represented by general formula (1) are not particularly limited, and any publicly known production method can be used.

In addition, cases where R¹ in a compound represented by general formula (1) is a group represented by general formula (2) are preferred because producing such a compound using any of Production Methods I to VI below is simple and inexpensive.

### Production Method I

A method comprising subjecting an alcohol compound represented by general formula (3) below and glycerin to a dehydrating condensation reaction.

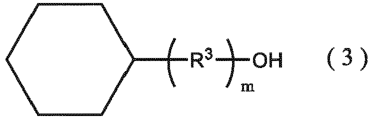

(In the formula, R³ denotes an alkylene group having 1 to 3 carbon atoms, and m represents a number that is 0 or 1.)

### Production Method II

A method comprising subjecting an alcohol compound represented by general formula (3) above and 1-chloro-2,3-propane diol to a dehydrochlorination reaction.

### Production Method III

A method comprising reacting an alcohol compound represented by general formula (3) with epichlorohydrin, and then hydrolyzing the thus obtained glycidyl ether compound.

### Production Method IV

A method comprising reacting an alcohol compound represented by general formula (3) with glycidol.

### Production Method V

A method comprising reacting an alcohol compound represented by general formula (3) with allyl chloride or allyl bromide, oxidizing using hydrogen peroxide or the like, and then hydrolyzing the thus obtained glycidyl ether compound.

### Production Method VI

A method comprising subjecting a compound represented by general formula (4) below and glycerin to a dehydrohalogenation reaction.

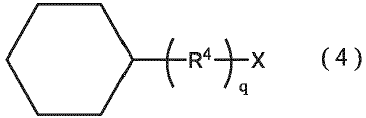

(In the formula, R⁴ denotes an alkylene group having 1 to 3 carbon atoms, q represents a number that is 0 or 1, and X denotes a halogen atom.)

Of the methods above, Production Method III is more preferred from the perspectives of being simple and inexpensive.

In general formula (3), R³ denotes an alkylene group having 1 to 3 carbon atoms, and specific examples thereof include a methylene group, an ethylene group, a propylene group and an isopropylene group. Of these, a methylene group or an ethylene group is preferred from the perspective of ease of preparation and procurement of raw materials. m denotes a number that is 0 or 1, and it is preferable for m to be 0 from the perspective of being able to obtain a compound that readily achieves the advantageous effect of this invention.

In general formula (4), R⁴ denotes an alkylene group having 1 to 3 carbon atoms, and specific examples thereof include methylene groups, ethylene groups, propylene groups and isopropylene groups. Of these, a methylene group or an ethylene group is preferred from the perspective of ease of preparation and procurement of raw materials. q denotes a number that is 0 or 1, and it is preferable for q to be 0 from the perspective of being able to obtain a compound that readily achieves the advantageous effect of this invention.

The blending quantity of a compound represented by general formula (1) is not particularly limited, but from the perspective of facilitating the advantageous effect of this invention, a compound represented by general formula (1) is preferably blended at a quantity of 0.05 to 40 mass%, more preferably 0.5 to 20 mass%, and further preferably 1 to 10 mass%, relative to the entire quantity of a composition containing water, the compound represented by general formula (1) and a sparingly water-soluble component.

The water used in this invention may be ordinary tap water or purified water. The usage quantity of water is not particularly limited, but from the perspective of facilitating the advantageous effect of this invention, water is more preferably present at a quantity of 40 to 99.9 mass%, further preferably 60 to 99 mass%, and even more preferably 80 to 98 mass%, relative to the entire quantity of a composition containing water, the compound represented by general formula (1) and a sparingly water-soluble component.

The sparingly water-soluble component used in this invention is not particularly limited as long as the component is highly oil-soluble and is sparingly soluble in water and has a solubility in water of 3 [g/100 g water] or less. However, from the perspective of facilitating the advantageous effect of this invention, the sparingly water-soluble component used in this invention is one or more components selected from the group consisting of sparingly water-soluble antibacterial/antiseptic agents, sparingly water-soluble antioxidants, sparingly water-soluble vitamins, sparingly water-soluble UV absorbers, sparingly water-soluble fragrances, sparingly water-soluble cosmetic components and sparingly water-soluble vegetable oils, and more preferably one or more components selected from the group consisting of sparingly water-soluble antibacterial/antiseptic agents, sparingly water-soluble UV absorbers, sparingly water-soluble vitamins and sparingly water-soluble fragrances.

Examples of sparingly water-soluble antibacterial/antiseptic agents include, but are not limited to, benzoic acid, salicylic acid, sorbic acid, para-oxybenzoic acid esters, para-chloro meta-cresol, hexachlorophene, chlorhexidine chloride, trichlorocarbanilide, phenoxyethanol, chlorphenesin, n-hexyl glyceryl ether, methylparaben, ethylparaben, butylparaben, caprylyl glycol, 2-ethylhexyl glyceryl ether, resorcin, triclosan, isopropylmethylphenol (IPMP), hinokitiol and phenol. Of these, it is preferable to use one or more components selected from the group consisting of n-hexyl glyceryl ether, phenoxyethanol, methylparaben, ethylparaben, butylparaben, caprylyl glycol and 2-ethylhexyl glyceryl ether from the perspective of facilitating the advantageous effect of this invention.

Examples of sparingly water-soluble antioxidants include, but are not limited to, dibutylhydroxytoluene, butylhydroxyanisole, sorbic acid, propyl gallate, gallic acid derivatives, ascorbic acid, ascorbic acid derivatives (ascorbic acid phosphate esters and the like), tocopherols, tocopherol derivatives, erythorbic acid, p-t-butylphenol and phytic acid. Of these, it is preferable to use one or more components selected from the group consisting of dibutylhydroxytoluene, tocopherols and tocopherol derivatives from the perspective of facilitating the advantageous effect of this invention.

Examples of sparingly water-soluble vitamins include vitamin A and derivatives thereof, vitamin B and derivatives thereof, vitamin C and derivatives thereof, vitamin D and derivatives thereof, vitamin E and derivatives thereof, vitamin F and derivatives thereof, and vitamin K and derivatives thereof, and specific examples thereof include, but are not limited to, stearyl ascorbate, ascorbyl dipalmitate, tocopherol nicotinate, menadione, dehydrocholesterol, ergocalciferol, pyridoxine dicaprylate, ascorbyl tetra-hexyldecanoate (VCIP), retinol, retinol palmitate, retinol acetate, docosahexaenoic acid, linoleic acid, pantenol, tocopherol linoleate, isopropyl linoleate, linolenic acid, pyridoxine palmitate, vitamin A oil, β-carotene, pyridoxine dipalmitate, phylloquinone, pantothenic acid and derivatives thereof, and biotin. Of these, it is preferable to use one or more components selected from the group consisting of vitamin A and derivatives thereof, vitamin C and derivatives thereof, and vitamin E and derivatives thereof from the perspective of facilitating the advantageous effect of this invention.

Examples of sparingly water-soluble UV absorbers include benzoic acid-based UV absorbers, anthranilic acid-based UV absorbers, salicylic acid-based UV absorbers, cinnamic acid-based UV absorbers, benzophenone-based UV absorbers, benzotriazole-based UV absorbers, triazine-based UV absorbers, benzoate-based UV absorbers, cyanoacrylate-based UV absorbers, oxanilide-based UV absorbers and formamidine-based UV absorbers.

Examples of benzoic acid-based UV absorbers include para-aminobenzoic acid, ethyl para-aminobenzoate, ethylhexyl para-dimethylaminobenzoate, octyl para-dimethylaminobenzoate, amyl para-dimethylaminobenzoate, monoglyceryl para-aminobenzoate, glyceryl para-aminobenzoate, ethyldihydroxypropyl para-aminobenzoate, ethyl N,N-dipropoxy-para-aminobenzoate, ethyl N,N-diethoxy-para-aminobenzoate, ethyl N,N-dimethyl-para-aminobenzoate, butyl N,N-dimethyl-para-aminobenzoate, amyl N,N-dimethyl-para-aminobenzoate, octyl N,N-dimethyl-para-aminobenzoate and hexyl diethylaminohydroxybenzoylbenzoate. Examples of anthranilic acid-based UV absorbers include homomenthyl-N-acetyl anthranilate.

Examples of salicylic acid-based UV absorbers include salicylic acid and sodium salts thereof, amyl salicylate, menthyl salicylate, homomenthyl salicylate, octyl salicylate, phenyl salicylate, benzyl salicylate and p-isopropanolphenyl salicylate. Examples of cinnamic acid-based UV absorbers include octyl cinnamate, ethyl 4-isopropylcinnamate, methyl 2,5-diisopropylcinnamate, ethyl 2,4-diisopropylcinnamate, methyl 2,4-diisopropylcinnamate, propyl p-methoxycinnamate, isopropyl p-methoxycinnamate, isoamyl p-methoxycinnamate, 2-ethylhexyl p-methoxycinnamate (2-ethylhexyl para-methoxycinnamate), 2-ethoxyethyl p-methoxycinnamate (cinoxate), cyclohexyl p-methoxycinnamate, ethyl α-cyano-β-phenylcinnamate, 2-ethylhexyl α-cyano-β-phenyl cinnamate (octocrylene), glyceryl mono-2-ethylhexanoyl-di-paramethoxycinnamate, ferulic acid and derivatives thereof.

Examples of benzophenone-based UV absorbers include 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone (oxybenzone-3), 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone and 5,5'-methylene-bis(2-hydroxy-4-methoxybenzophenone).

Examples of benzotriazole-based UV absorbers include 2-(2-hydroxy-5-methylphenyl)benzotriazole, 2-(2-hydroxy-5-methylphenyl)-5-chlorobenzotriazole, 2-(2-hydroxy-5-tert-octylphenyl)benzotriazole, 2-(2-hydroxy-5-tert-octylphenyl)-5-chlorobenzotriazole, 2-(2-hydroxy-3,5-di-tert-butylphenyl)benzotriazole, 2-(2-hydroxy-3,5-di-tert-butylphenyl)-5-chlorobenzotriazole, 2-(2-hydroxy-3-tert-butyl-5-methylphenyl)benzotriazole, 2-(2-hydroxy-3-tert-butyl-5-methylphenyl)-5-chlorobenzotriazole, 2-(2-hydroxy-3,5-dicumylphenyl)benzotriazole, 2-(2-hydroxy-3,5-dicumylphenyl)-5-chlorobenzotriazole, 2,2'-methylene-bis(4-tert-octyl-6-benzotriazolylphenol), polyethylene glycol esters of 2-(2-hydroxy-3-tert-butyl-5-carboxyphenyl)benzotriazole, 2-[2-hydroxy-3-(2-acryloyloxyethyl)-5-methylphenyl]benzotriazole, 2-[2-hydroxy-3-(2-acryloyloxyethyl)-5-methylphenyl]-5-chlorobenzotriazole, 2-[2-hydroxy-3-(2-methacryloyloxyethyl)-5-tert-butylphenyl]benzotriazole, 2-[2-hydroxy-3-(2-methacryloyloxyethyl)-5-tert-butylphenyl]-5-chlorobenzotriazole, 2-[2-hydroxy-3-(2-methacryloyloxyethyl)-5-tert-octylphenyl]benzotriazole, 2-[2-hydroxy-3-(2-methacryloyloxyethyl)-5-tert-octylphenyl]-5-chlorobenzotriazole, 2-[2-hydroxy-3-(2-methacryloyloxyethyl)-5-tert-butylphenyl]benzotriazole, 2-[2-hydroxy-3-(2-methacryloyloxyethyl)-5-tert-butylphenyl]-5-chlorobenzotriazole, 2-[2-hydroxy-5-(2-methacryloyloxyethyl)phenyl]benzotriazole, 2-[2-hydroxy-5-(2-methacryloyloxyethyl)phenyl]-5-chlorobenzotriazole, 2-[2-hydroxy-3-tert-butyl-5-(2-methacryloyloxyethyl)phenyl]benzotriazole, 2-[2-hydroxy-3-tert-butyl-5-(2-methacryloyloxyethyl)phenyl]-5-chlorobenzotriazole, 2-[2-hydroxy-3-tert-amyl-5-(2-methacryloyloxyethyl)phenyl]benzotriazole, 2-[2-hydroxy-3-tert-amyl-5-(2-methacryloyloxyethyl)phenyl]-5-chlorobenzotriazole, 2-[2-hydroxy-3-tert-butyl-5-(3-methacryloyloxypropyl)phenyl]benzotriazole, 2-[2-hydroxy-3-tert-butyl-5-(3-methacryloyloxypropyl)phenyl]-5-chlorobenzotriazole, 2-[2-hydroxy-4-(2-methacryloyloxymethyl)phenyl]benzotriazole, 2-[2-hydroxy-4-(2-methacryloyloxymethyl)phenyl]-5-chlorobenzotriazole, 2-[2-hydroxy-4-(3-methacryloyloxy-2-hydroxypropyl)phenyl]benzotriazole, 2-[2-hydroxy-4-(3-methacryloyloxy-2-hydroxypropyl)phenyl]-5-chlorobenzotriazole, 2-[2-hydroxy-4-(3-methacryloyloxypropyl)phenyl]benzotriazole and 2-[2-hydroxy-4-(3-methacryloyloxypropyl)phenyl]-5-chlorobenzotriazole.

Examples of triazine-based UV absorbers include 2,4,6-tris[4-(2-ethylhexyloxycarbonyl)anilino]-1,3,5-triazine, 2,4-bis-[{4-(2-ethylhexyloxy)-2-hydroxy}-phenyl]-6-(4-methoxyphenyl)-1,3,5-triazine, 2-(2-hydroxy-4-methoxyphenyl)-4,6-diphenyl-1,3,5-triazine, 2-(2-hydroxy-4-hexyloxyphenyl)-4,6-diphenyl-1,3,5-triazine, 2-(2-hydroxy-4-octoxyphenyl)-4,6-bis(2,4-dimethylphenyl)-1,3,5-triazine, 2-[2-hydroxy-4-(3-C₁₂-C₁₃ mixed alkoxy-2-hydroxypropoxy)phenyl]-4,6-bis(2,4-dimethylphenyl)-1,3,5-triazine, 2-[2-hydroxy-4-(2-acryloyloxyethoxy)phenyl]-4,6-bis(4-methylphenyl)-1,3,5-triazine, 2-[2-hydroxy-4-(2-acetyloxyethoxy)phenyl]-4,6-bisphenyl-1,3,5-triazine, 2-(2,4-dihydroxy-3-allylphenyl)-4,6-bis(2,4-dimethylphenyl)-1,3,5-triazine and 2,4,6-tris(2-hydroxy-3-methyl-4-hexyloxyphenyl)-1,3,5-triazine. Examples of benzoate-based UV absorbers include resorcinol monobenzoate, 2,4-di-tert-butylphenyl-3,5-di-tert-butyl-4-hydroxybenzoate, octyl (3,5-di-tert-butyl-4-hydroxy)benzoate, dodecyl (3,5-di-tert-butyl-4-hydroxy)benzoate, tetradecyl (3,5-di-tert-butyl-4-hydroxy)benzoate, hexadecyl (3,5-di-tert-butyl-4-hydroxy)benzoate, octadecyl (3,5-di-tert-butyl-4-hydroxy)benzoate, behenyl (3,5-di-tert-butyl-4-hydroxy)benzoate and stearyl (3,5-di-tert-butyl-4-hydroxy)benzoate.

Examples of cyanoacrylate-based UV absorbers include ethyl-α-cyano-β,β-diphenyl acrylate and methyl-2-cyano-3-methyl-3-(p-methoxyphenyl) acrylate. Examples of oxanilide-based UV absorbers include 2-ethyl-2'-ethoxyoxanilide and 2-ethoxy-4'-dodecyloxanilide. Examples of formamidine-based UV absorbers include N,N'-diphenyl-N'-(4-ethoxycarbonylphenyl)formamidine, N'-(4-ethoxycarbonylphenyl)-N-methyl-N-phenylformamidine, N,N'-bis(4-ethoxycarbonylphenyl)-N-methylformamidine, N'-(4-ethoxycarbonylphenyl)-N-(2'-methoxyphenyl)-N-methylformamidine and N-(4-n-butoxycarbonylphenyl)-N'-(4'-ethylcarbonyl)-N-methylformamidine.

Examples of other UV absorbers include, but are not limited to, 3-(4'-methylbenzylidene)-d,l-camphor, 3-benzylidene-d,l-camphor, 2-phenyl-5-methylbenzoxazole, dibenzalazine, dianisoylmethane, 5-(3,3-dimethyl-2-norbornilidene)-3-pentan-2-one, 4-t-butylmethoxydibenzoylmethane, octyl triazone, urocanic acid, ethyl urocanate, 1-(3,4-dimethoxyphenyl)-4,4-dimethyl-1,3-pentane dione, 2-ethylhexyl dimethoxybenzylidene dioxoimidazolidine propionate, phenylbenzimidazole sulfonic acid, terephthalylidine dicamphor sulfonic acid, drometrizole trisiloxane, methyl anthranilate, rutin, rutin derivatives, oryzanol and oryzanol derivatives. Of these, it is preferable to use one or more components selected from the group consisting of benzoic acid-based UV absorbers, cinnamic acid-based UV absorbers, benzophenone-based UV absorbers and benzotriazole-based UV absorbers from the perspective of facilitating the advantageous effect of this invention.

Sparingly water-soluble fragrances are substances in which compound perfumes containing natural fragrances and/or synthetic fragrances are used as flavor bases and these are dissolved in vegetable oils or the like. With regard to natural fragrances and synthetic fragrances that serve as flavor bases of sparingly water-soluble fragrances, examples of natural fragrances include amyris oil, ambrette seed oil, ylang ylang oil, ylang ylang absolute, iris resinoid, iris absolute, iris oil, wintergreen oil, estragon oil, elemi oleoresin, elemi resinoid absolute, elemi tincture, oakmoss concrete, oakmoss absolute, oakmoss resin, oakmoss resinoid, osmanthus absolute, osmanthus concrete, opopanax resinoid, opopanax absolute, opopanax oil, olibanum resinoid, olibanum absolute, olibanum oil, all spice oil, origanum oil, oregano oil, oregano oleoresin, orange flower absolute, orange flower concrete, kananga oil, gurjun balsam, gurjun balsam oil, cassie absolute, cassie flower oil, cassia oil, gardenia absolute, carnation absolute, cabreuva oil, chamomile oil, cardamom oil, galbanum oil, galbanum resin, galbanum resinoid, caraway seed oil, carrot seed oil, cubeba oil, guaicum wood oil, guaicum resin, guaicum concrete, cinnamomum camphora oil, cumin oil, cumin absolute, cumin oleoresin, clary sage oil, grapefruit oil, clove oil, costus oil, copaiba balsam, copaiba balsam oil, copaiba balsam resin, coriander oil, sandalwood oil, perilla oil, cedarwood oil, citronella oil, jasmine oil, jasmine absolute, jasmine concrete, juniper berry oil, genet absolute, jonquil absolute, ginger oil, cinnamon oil, cinnamon bark oil, cinnamon leaf oil, Japanese cedar oil, star anise oil, styrax oil, styrax resinoid, spike lavender oil, spearmint oil, savory oil, sage oil, cedar oil, cedar leaf oil, geranium oil, celery seed oil, thyme oil, taguette oil, tangerine oil, tuberose absolute, tea tree oil, tree moss absolute, tonka bean oil, true balsam, nutmeg oil, narcissus absolute, neroli oil, violet leaf absolute, pine oil, pine needle oil, basil oil, parsley leaf oil, parsley seed oil, parsley herb oil, patchouli oil, peppermint oil, vanilla absolute, honeysuckle absolute, palmarosa oil, valerian oil, bitter orange oil, hyssop oil, Japanese cypress oil, white cedar oil, hyacinth absolute, fennel oil, fig absolute, petitgrain oil, buchu oil, bay oil, vetiver oil, pepper oil, peppermint absolute, peppermint oil, bergamot oil, Peru balsam, benzoin tincture, benzoin resinoid, Cinnamomum camphora oil, marjoram oil, mandarin oil, tangerine oil, mimosa concrete, mimosa absolute, mimosa oil, mill resinoid, mill absolute, mill oil, musk absolute, musk tincture, eucalyptus oil, yuzu oil, lime oil, labdanum oil, labdanum resinoid, lavender oil, lavender absolute, Lavandula burnatii oil, Lavandula burnatii absolute, lemon oil, lemongrass oil, rose oil, rose absolute, rose concrete, rosemary oil, laurel oil and laurel leaf oil.

Examples of synthetic fragrances include, but are not limited to, ambrettolide, C₆-C₁₂ aldehydes, anisic aldehyde, acetal R, acetophenone, acetyl cedrene, adoxal, allyl amyl glycolate, allyl cyclohexanepropionate, ambroxan, amyl cinnamaldehyde, amyl cinnamaldehyde dimethyl acetal, amyl valerianate, amyl salicylate, acetyl eugenol, isoamyl acetate, isoamyl salicylate, indole, ionone, isobornyl acetate, isocyclocitral, Iso E Super, isoeugenol, isononyl acetate, isobutylquinoline, γ-undecalactone, ethylene brassylate, ethylene dodecanedioate, ethylvanillin, 2-ethylhexanol, aurantiol, 10-oxahexadecanolide, 11-oxahexadecanolide, 12-oxahexadecanolide, oxahexadecen-2-one, eugenol, orivone, oxyphenylone, galaxolide, caryophyllene, cashmeran, carvone, β-caryophyllene, Calone, coumarin, p-cresyl methyl ether, geraniol, geranyl acetate, geranyl formate, geranyl nitrile, Koavone, Sandalore, Sandela, Santalex, cinnamic alcohol, cinnamaldehyde, cis-jasmon, citral, citral dimethyl acetal, citrasal, citronellal, citronellol, citronellyl acetate, citronellyl formate, citronellyl nitrile, cyclaset, cyclamen aldehyde, cyclaprop, dimethyl benzyl carbinol, dihydrojasmone, dihydrolinalool, dihydromyrcenol, Dimetol, dimyrcetol, diphenyl oxide, jasmal, jasmolactone, jasmophyllan, cinnamyl acetate, cyclopentadecanone, cyclohexadecenone, cyclopentadecanolide, cyclohexadecanolide, dimethyl benzyl carbinyl acetate, jasmacyclene, styrax acetate, styrax propionate, cedramber, cedryl acetate, cedrol, selestride, α-damascone, β-damascone, δ-damascone, damascenones, terpineol, terpinyl acetate, thymol, tetrahydrolinalool, tetrahydrolinalyl acetate, tetrahydrogeraniol, tetrahydrogeranyl acetate, tonalide, traseolide, Triplal, neryl acetate, nerol, neobergamate, γ-nonalactone nopyl alcohol, nopyl acetate, Bacdanol, hydrotropic alcohol, α-pinene, β-pinene, hydroxycitronellal, hyacinth dimethyl acetal, butyl butyrate, p-t-butylcyclohexanol, p-t-butylcyclohexyl acetate, o-t-butylcyclohexanol, o-t-butylcyclohexyl acetate, fruitate, phentyl alcohol, phenyl ethyl phenyl acetate, phenyl ethyl acetate, pentalide, verdox, benzyl acetate, benzyl alcohol, benzyl salicylate, bergamyl acetate, benzaldehyde, benzyl formate, hedione, helional, heliotropine, cis-3-hexenol, cis-3-hexenyl acetate, cis-3-hexenyl salicylate, hexylcinnamaldehyde, hexyl salicylate, bornyl acetate, borneol, manzanate, Mayol, myrcene, myrac aldehyde, muguet aldehyde, mugol, musk TM-11, musk 781, musk C14, muscone, musk ketone, musk tibetine, menthanyl acetate, menthonate, methyl anthranilate, methyl eugenol, menthol, α-methylionone, β-methylionone, γ-methylionone, methyl isoeugenol, methyl lavender ketone, methyl salicylate, 14-methyl-hexadecenolide, 14-methyl-hexadecanolide, methyl naphthyl ketone, methyl phenyl acetate, yara yara, δ-C₆₋₁₃ lactones, lime oxide, γ-C₆₋₁₃ lactones, raspberry ketone, limonene, ligustral, lilial, linalool, linalool oxide, linalyl acetate, lyral, rhubafuran, rosephenone, rose oxide and vanillin.

Examples of sparingly water-soluble cosmetic components include, but are not limited to, sparingly water-soluble placenta extract liquids, sparingly water-soluble mulberry bark extracts, sparingly water-soluble meadow saxifrage extracts, sparingly water-soluble perilla extracts, sparingly water-soluble white mustard extracts, sparingly water-soluble damask rose extracts, sparingly water-soluble Chinese peony extracts, sparingly water-soluble lotus seed extracts, sparingly water-soluble Codonopsis pilosul extracts, sparingly water-soluble pearl barley hydrolyzates, sparingly water-soluble Pandanus Amaryllifolius extracts, sparingly water-soluble Arcangelicia flava extracts, sparingly water-soluble kiwi extracts, sparingly water-soluble Matricaria chamomilla extracts, sparingly water-soluble common glasswort extracts, sparingly water-soluble Oryza sativa leaf extracts, sparingly water-soluble eggplant (water eggplant, long eggplant, kamo eggplant, rice eggplant) extracts, sparingly water-soluble seaweed extracts, sparingly water-soluble extracts of marine phonerogram plants, sparingly water-soluble rice fermentation extracts, linoleic acid, liposomal linoleic acid, sparingly water-soluble animal-derived and fish-derived collagen and derivatives thereof, sparingly water-soluble elastin and derivatives thereof, sparingly water-soluble glycyrrhizinic acid and derivatives thereof, sparingly water-soluble t-cycloamino acid derivatives, allantoin, arbutin, sparingly water-soluble Gentiana extracts, sparingly water-soluble licorice extracts, sparingly water-soluble carrot extracts, sparingly water-soluble aloe extracts, sparingly water-soluble Laminaria angastata extracts, sparingly water-soluble Ulva pertusa extracts, sparingly water-soluble Rhamnoceae Zizyphus joazeiro extracts and sparingly water-soluble peach extracts.

Examples of sparingly water-soluble vegetable oils include, but are not limited to, rosemary oil, Matricaria chamomilla oil, eucalyptus oil, rice germ oil, wheat germ oil, γ-oryzanol, plant ceramides (glycosylceramides), carrot oil, sparingly water-soluble coix seed extracts, sparingly water-soluble field horsetail extracts, sparingly water-soluble arnica extracts, sparingly water-soluble chamomile extracts, sparingly water-soluble Lithospermi Radix extracts, sparingly water-soluble Tilia japonica extracts, sparingly water-soluble Achillea millefolium extracts, sparingly water-soluble sage extracts, sparingly water-soluble Angelica acutiloba extracts, sparingly water-soluble horse chestnut extracts, sparingly water-soluble peach leaf extracts, sparingly water-soluble rosemary extracts, sparingly water-soluble pearl barley extracts, olive oil, sparingly water-soluble loquat extracts, borage oil, camellia oil and evening primrose oil.

The blending quantity of these sparingly water-soluble components is not particularly limited, but from the perspective of facilitating the advantageous effect of this invention, these sparingly water-soluble components are preferably blended at a quantity of 0.05 to 40 mass%, more preferably 0.5 to 20 mass%, and further preferably 1 to 10 mass%, relative to the entire quantity of a composition containing water, a compound represented by general formula (1) and the sparingly water-soluble components.

This invention is a method in which a compound represented by general formula (1) improves the solubility of a sparingly water-soluble component in a system in which the compound represented by general formula (1), water and the sparingly water-soluble component are present. The blending ratio of the compound represented by general formula (1) and the sparingly water-soluble component is not particularly limited, but there may be cases in which it is necessary to increase the usage quantity of the compound represented by general formula (1) as the solubility of a sparingly water-soluble component decreases. Within this scope, the blending ratio of the compound represented by general formula (1) and the sparingly water-soluble component is preferably such that the (compound represented by general formula (1)):(sparingly water-soluble component) mass ratio is 1:0.05 to 1:5 from the perspective of better realizing the advantageous effect of this invention.

Furthermore, by blending an alcohol compound in addition to a compound represented by general formula (1), it is possible to further improve the solubility of a sparingly water-soluble component. Examples of alcohol compounds include ethanol, propanol, isopropanol, butanol, propylene glycol, dipropylene glycol, butylene glycol and glycerin, and among these, butanol, propylene glycol, dipropylene glycol, butylene glycol and glycerin are preferred, with propylene glycol and butylene glycol being more preferred and butylene glycol being most preferred, from the perspective of better realizing the advantageous effect of this invention.

The blending quantity of the alcohol compound is not particularly limited, but from the perspective of facilitating the advantageous effect of this invention, the alcohol compound is preferably blended at a quantity of 0.05 to 40 mass%, more preferably 0.5 to 20 mass%, and further preferably 1 to 10 mass%, relative to the entire quantity of a composition containing water, a compound represented by general formula (1), a sparingly water-soluble component and the alcohol compound.

In addition, the blending ratio of a compound represented by general formula (1) and an alcohol compound is not particularly limited, but from the perspective of better realizing the advantageous effect of this invention, the blending ratio of a compound represented by general formula (1) and an alcohol compound is preferably such that the (compound represented by general formula (1)):(alcohol compound) mass ratio is 1:0.5 to 1:5.

When carrying out the method of this invention, applications thereof are not limited, and the method of this invention can also be used in any application in a system which contains a compound represented by general formula (1), water and a sparingly water-soluble component, which are essential components in this invention, and which includes a step in which an improvement in the solubility of the sparingly water-soluble component is required. Of these, applications in the technical field of cosmetic products are preferred, with applications in the technical field of water-based cosmetic products being more preferred, due to a large number of cases in which the advantageous effect mentioned above can be expected.

Here, the term "water-based cosmetic product" in the present specification means a cosmetic product in which water and water soluble components account for 60% or more of all components in the cosmetic product, and also includes O/W type emulsions. In addition, the formulation type is not particularly limited, and examples thereof include toners, lotions, milky lotions, serums, gels creams and essences. Among water-based cosmetic products, use in applications in which usage quantities of surfactants such as well-known emulsifying agents and solubilizing agents for cosmetic products are restricted is particularly preferred. The reason for this is that if a water-based cosmetic product is produced by means of the process for improving the solubility of a sparingly water-soluble component of this invention, it is possible to blend the sparingly water-soluble component in the water-based cosmetic product and impart the water-based cosmetic product with high functionality using less or no surfactant such as an emulsifying agent or a solubilizing agent.

When producing a water-based cosmetic product, it is possible to blend optional components that are commonly used as additives for cosmetic products in addition to a compound represented by general formula (1), water and a sparingly water-soluble component, which are essential components in this invention. However, when producing a water-based cosmetic product, essential conditions are that the process for improving the solubility of a sparingly water-soluble component of this invention is included and that qualitative and quantitative ranges thereof are satisfied so that the advantageous effect of this invention is not impaired.

### Examples

This invention will now be explained in detail by means of examples, but this invention is in no way limited to these examples, and may be altered as long as such alterations do not deviate from the scope of this invention. Moreover, in the examples etc. given below, % means mass percentage unless explicitly indicated otherwise.

Compounds that correspond to compounds represented by general formula (1), which are used in the examples, are shown below.

### <Compounds represented by general formula (1)>

Compound (1)-2: Cyclohexyl glyceryl ether (a compound in which R¹ in general formula (1) is a group represented by general formula (2) and n is 0 in general formula (2))

Compounds that replace compounds represented by general formula (1), which are used in the comparative examples, are shown below. Compound (1)-1: 1,2-hexane diol (a compound in which R1 in general formula (1) is a propyl group)

### <Highly water-soluble hydroxyl group-containing compounds>

Ethanol
Propylene glycol
Dipropylene glycol
1,3-butylene glycol
Glycerin

Moreover, examples of compounds similar to compounds represented by general formula (1) include 1,2-heptane diol, n-hexyl glyceryl ether and 2-ethylhexyl glyceryl ether. However, these compounds naturally exhibit poor solubility in water as compounds per se, and are therefore not suitable as comparative examples of compounds represented by general formula (1), and it is not possible to carry out investigations into improvements in solubility of the sparingly water-soluble components listed below. Therefore, these are excluded from comparative products.

Sparingly water-soluble compounds used in examples and comparative examples are shown below. Moreover, compounds having a solubility in water of 3 [g/100 g water] or less are used.

### <Sparingly water-soluble components>

n-hexyl glyceryl ether (antibacterial/antiseptic agent) Methylparaben (antibacterial/antiseptic agent)
Caprylyl glycol (antibacterial/antiseptic agent)
2-ethylhexyl glyceryl ether (antibacterial/antiseptic agent) Phenoxyethanol (antibacterial/antiseptic agent)
Tocopherols (vitamins)
Peppermint oil (fragrance)

### [Investigations into improvements in solubility of sparingly water-soluble components]

First, Solutions 1 to 9 shown in Table 1 below were prepared. Units for the numerical values shown in Table 1 are [g], and Solutions 1 to 9 were transparent colorless solutions.

**Table 1**

| 1 | Solution 1 | Solution 2 | Solution 3 | Solution 4 | Solution 5 | Solution 6 | Solution 7 | Solution 8 | Solution 9 |
|---|---|---|---|---|---|---|---|---|---|
| Water | 100 | 90 | 90 | 90 | 90 | 90 | 90 | 90 | 80 |
| Compound (1)-1 | | 10 | | | | | | | |
| Compound (1)-2 | | | 10 | | | | | | 10 |
| Ethanol | | | | 10 | | | | | |
| Propylene glycol | | | | | 10 | | | | |
| Dipropylene glycol | | | | | | 10 | | | |
| Butylene glycol | | | | | | | 10 | | 10 |
| Glycerin | | | | | | | | 10 | |

Next, solubility [g/100 g solution] in Solutions 1 to 9 was investigated for three sparingly water-soluble components whose solubility in water could be confirmed but whose solubility was 1 [g/100 g water] or less (methylparaben, caprylyl glycol and 2-ethylhexyl glyceryl ether). In terms of test procedure, a target sparingly water-soluble component was added 0.1 g at a time to each of Solutions 1 to 9 shown in Table 1, and the added quantity of the sparingly water-soluble component immediately before the added quantity at which turbidity occurred is shown as the solubility in the table. In addition, confirmation that turbidity or precipitation had not occurred was carried out by adding 0.1 g of the sparingly water-soluble component at a time, stirring for 10 minutes at 25°C and then leaving to stand. For example, in a case where methylparaben was dissolved in Solution 1, complete dissolution occurred until 0.2 g of methylparaben had been added, and turbidity occurred at the stage where 0.3 g of methylparaben had been added, and the solubility was therefore taken to be 0.2 g. Moreover, further tests were not carried out in cases where the solubility exceeded 5 [g/100 g solution].

Units for the numerical values shown in Table 2 below are [g/100 g solution].

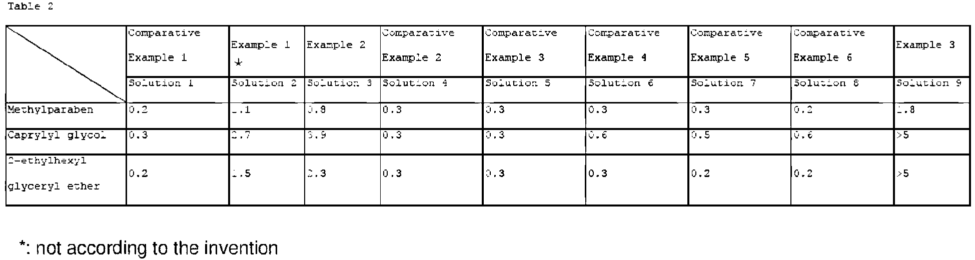

As a result, by comparing Comparative Example 1 with Examples 1 and 2, it was understood that the solubility of all three of these sparingly water-soluble components (methylparaben, caprylyl glycol and 2-ethylhexyl glyceryl ether) improved in Solution 2 and Solution 3, in which Compound (1)-1 (not according to the invention) and Compound (1)-2 were used. In addition, it was confirmed that the solubility of these sparingly water-soluble components was further improved in Solution 9, in which Compound (1)-2 and butylene glycol were both used.

Next, in order to investigate whether or not a similar advantageous effect was achieved for other sparingly water-soluble components (n-hexyl glyceryl ether, phenoxyethanol, tocopherols and peppermint oil), solubility values [g/100 g solution] were investigated by carrying out similar tests to those described above using Solutions 1, 2, 3 and 8.

**Table 3**

| | Comparative Example 7 | Example 4 * | Example 5 | Comparative Example 8 |
|---|---|---|---|---|
| | Solution 1 | Solution 2 | Solution 3 | Solution 8 |
| n-hexyl glyceryl ether | 1.0 | >5 | >5 | 1.2 |
| Phenoxyethanol | 2.7 | 3.9 | 3.9 | 2.7 |
| Tocopherols | Insoluble | 0.8 | 0.2 | Insoluble |
| Peppermint oil | Insoluble | 0.5 | 0.3 | Insoluble |

| | | | | |
|---|---|---|---|---|
| (*: not according to the invention) | | | | |

As a result, it became clear that the solubility of these other sparingly water-soluble components was improved in the same way as in the tests carried out using methylparaben, caprylyl glycol and 2-ethylhexyl glyceryl ether, which are also sparingly water-soluble components, in Solution 2, Solution 3 and Solution 9, in which Compound (1)-1 and Compound (1)-2 were used. In particular, it was confirmed that solubility in water of tocopherols and peppermint oil was achieved by using Compound (1)-1 (not according to the invention) and Compound (1)-2, despite tocopherols and peppermint oil being insoluble components that do not dissolve in water at all.

Moreover, formulations containing the sparingly water-soluble components obtained in Examples 1 to 5 can be used as transparent cosmetic products that impart functions (functionality) exhibited by sparingly water-soluble components, and it is also possible to blend optional components that are commonly used as additives for cosmetic products. As a specific example, Table 4 shows a formulation example of a transparent cosmetic product formulated using the method of this invention.

### - Formulation example 1 (a transparent cosmetic product)

**Table 4**

| Component | Blending quantity (mass%) |
|---|---|
| Compound (1)-2 | 10 |
| Tocopherols | 0.2 |
| Betaine | 1 |
| 1% aqueous solution of hyaluronic acid | 2 |
| Water | Balance |
| Total | 100 |

As a result, by using the method of this invention, it is possible to obtain a highly functional transparent cosmetic product which is gentle on the skin and which contains tocopherols (vitamins) that are insoluble in water without using a surfactant.

### Industrial Applicability

The method of this invention is not limited in terms of application, and can also be used in any type of application as long as this is an application that requires an improvement in the solubility of a sparingly water-soluble component. Of these applications, a high degree of functionalization of water-based cosmetic products by sparingly water-soluble components can be realized in water-based cosmetic product applications in which usage quantities of surfactants such as emulsifying agents and solubilizing agents are restricted, and this invention is therefore extremely useful.

## Claims

1. A method for improving solubility of a sparingly water-soluble component, the method comprising blending a compound represented by general formula (1) below with the sparingly water-soluble component in the presence of water, wherein the sparingly water-soluble component has a solubility in water of 3[g/100g water] or less, and wherein the sparingly water-soluble component is one or more components selected from the group consisting of sparingly water-soluble antibacterial/antiseptic agents, sparingly water-soluble antioxidants, sparingly water-soluble vitamins, sparingly water-soluble UV absorbers, sparingly water-soluble fragrances, sparingly water-soluble cosmetic components and sparingly water-soluble vegetable oils, 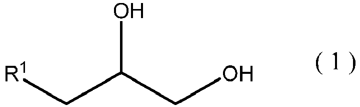 wherein R¹ denotes a group represented by general formula (2) ; 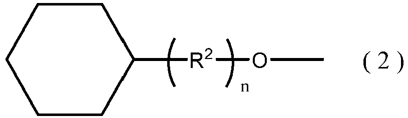 wherein R² denotes an alkylene group having 1 to 3 carbon atoms, and n represents a number that is 0 or 1.

2. The method of claim 1, wherein the compound represented by general formula (1) is blended at a quantity of 0.05 to 40 mass% relative to the total quantity of a composition containing water, the compound represented by general formula (1) and the sparingly water-soluble component.

3. The method of any one of claims 1 to 2, wherein the sparingly water-soluble component is blended at a quantity of 0.05 to 40 mass% relative to the total quantity of a composition containing water, the compound represented by general formula (1) and the sparingly water-soluble component.

4. The method of any one of claims 1 to 3, which further comprises blending an alcohol compound.

5. The method of any one of claims 1 to 3, in which a surfactant is not used.

6. A method for producing a water-based cosmetic product, comprising using the method of any one of claims 1 to 5.

## Patentansprüche

1. Verfahren zum Verbessern der Löslichkeit einer schwer wasserlöslichen Komponente, wobei das Verfahren das Mischen einer allgemein durch die folgende Formel (1) wiedergegebenen Verbindung mit der schwer wasserlöslichen Komponente in Anwesenheit von Wasser umfasst, wobei die schwer wasserlösliche Komponente eine Löslichkeit in Wasser von 3 g/100 g Wasser oder weniger aufweist, und wobei die schwer wasserlösliche Komponente aus einer oder mehreren Komponenten aus der Gruppe besteht, die schwer wasserlösliche antibakterielle/antiseptische Mittel, schwer wasserlösliche Antioxidantien, schwer wasserlösliche Vitamine, schwer wasserlösliche UV-Absorber, schwer wasserlösliche Duftstoffe, schwer wasserlösliche kosmetische Komponenten und schwer wasserlösliche Pflanzenöle umfasst, 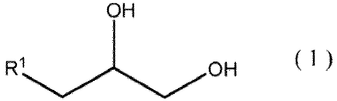 wobei R¹ eine durch die allgemeine Formel (2) wiedergegebene Gruppe angibt, 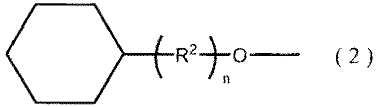 wobei R² eine Alkylengruppe mit 1 bis 3 Kohlenstoffatomen angibt und n eine Zahl von 0 oder 1 wiedergibt.

2. Verfahren nach Anspruch 1, wobei die durch die allgemeine Formel (1) wiedergegebene Verbindung mit einem Anteil von 0,05 bis 40 Massenprozent relativ zu der Gesamtmenge einer Wasser enthaltenden Zusammensetzung beigemischt wird, wobei die Verbindung durch die allgemeine Formel (1) und die schwer wasserlösliche Komponente wiedergegeben wird.

3. Verfahren nach Anspruch 1 oder 2, wobei die schwer wasserlösliche Komponente mit einem Anteil von 0,05 bis 40 Massenprozent relativ zu der Gesamtmenge einer Wasser enthaltenden Zusammensetzung beigemischt wird, wobei die Verbindung durch die allgemeine Formel (1) und die schwer wasserlösliche Komponente wiedergegeben wird.

4. Verfahren nach einem der Ansprüche 1 bis 3, das weiterhin das Beimischen einer Alkoholverbindung umfasst.

5. Verfahren nach einem der Ansprüche 1 bis 3, wobei kein Tensid verwendet wird.

6. Verfahren zum Erzeugen eines wasserbasierten kosmetischen Produkts, das die Verwendung des Verfahrens gemäß einem der Ansprüche 1 bis 5 umfasst.

## Revendications

1. Procédé pour améliorer la solubilité d'un composant peu soluble dans l'eau, le procédé comprenant le mélange d'un composé représenté par la formule générale (1) ci-dessous avec le composant peu soluble dans l'eau en présence d'eau, dans lequel le composant peu soluble dans l'eau a une solubilité dans l'eau de 3 [g/100 g d'eau] ou moins, et dans lequel le composant peu soluble dans l'eau est un ou plusieurs composants choisis dans le groupe constitué par les agents antibactériens/antiseptiques peu solubles dans l'eau, les antioxydants peu solubles dans l'eau, les vitamines peu solubles dans l'eau, les absorbeurs d'UV peu solubles dans l'eau, les parfums peu solubles dans l'eau, les composants cosmétiques peu solubles dans l'eau et les huiles végétales peu solubles dans l'eau, 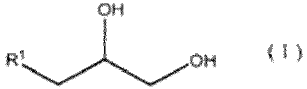 R¹ désignant un groupe représenté par la formule générale 2 ; 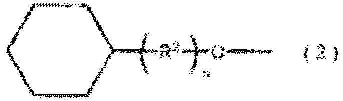 R² désignant un groupe alkylène ayant de 1 à 3 atomes de carbone, et n représentant un nombre égal à 0 ou 1.

2. Procédé selon la revendication 1, dans lequel le composé représenté par la formule générale (1) est mélangé en une quantité de 0,05 à 40 % en masse par rapport à la quantité totale d'une composition contenant de l'eau, le composé représenté par la formule générale (1) et le composant peu soluble dans l'eau.

3. Procédé selon l'une quelconque des revendications 1 à 2, dans lequel le composant peu soluble dans l'eau est mélangé en une quantité de 0,05 à 40 % en masse, par rapport à la quantité totale d'une composition contenant de l'eau, le composé représenté par la formule générale (1) et le composant peu soluble dans l'eau.

4. Procédé selon l'une quelconque des revendications 1 à 3, qui comprend en outre le mélange d'un composé alcool.

5. Procédé selon l'une quelconque des revendications 1 à 3, dans lequel un tensioactif n'est pas utilisé.

6. Procédé de fabrication d'un produit cosmétique à base d'eau, comprenant l'utilisation du procédé selon l'une quelconque des revendications 1 à 5.
